# FASCICULE DE BREVET EUROPEEN

(11) **EP 2 032 514 B1**
(45) Date de publication et mention de la délivrance du brevet: **01.05.2013**
(21) Numéro de dépôt: 07803790.0
(22) Date de dépôt: 27.06.2007
(51) Int. Cl.: C07C 37/84, C07C 39/08

(54) **PROCÉDÉ DE PRÉPARATION D'HYDROQUINONE PURIFIÉE**
VERFAHREN ZUR HERSTELLUNG VON GEREINIGTEM HYDROCHINON
PROCESS FOR PREPARING PURIFIED HYDROQUINONE

(30) Priorité: 29.06.2006 FR 0605872
(43) Date de publication de la demande: 11.03.2009
(73) Titulaire: Rhodia Opérations, 93306 Aubervilliers (FR)
(72) Inventeur: MASSON, Jean-Claude, F-69003 Lyon (FR); CARVIN, Philippe, F-69005 Lyon (FR); GRIENEISEN, Jean-Louis, F-69960 Corbas (FR)
(74) Mandataire: Dutruc-Rosset, Marie-Claude
(86) Numéro de dépôt international: PCT/FR2007/001081
(87) Numéro de publication internationale: WO 2008/000957

(56) Documents cités:
- FR-A1- 2 467 185
- PUEL F ET AL: "Simulation and analysis of industrial crystallization processes through multidimensional population balance equations. Part 2: a study of semi-batch crystallization" CHEMICAL ENGINEERING SCIENCE, OXFORD, GB, vol. 58, no. 16, août 2003 (2003-08), pages 3729-3740, XP004442727 ISSN: 0009-2509
- "Hydroquinone, Resorcinol, and Catechol" In: Kirk-Othmer: "Encyclopedia of chemical technology, fourth edition", 1995, John Wiley and sons, New york vol. 13, pages 996-1002,

## Description

La présente invention a pour objet un procédé de préparation d'hydroquinone purifiée.

L'invention vise à fournir l'hydroquinone débarrassée des impuretés résultantes de son procédé de préparation.

Selon un mode de réalisation, l'invention fournit un procédé permettant d'obtenir de l'hydroquinone de grande pureté.

L'hydroquinone (ou 1,4-dihydroxybenzène) est un produit largement utilisé dans de nombreux domaines d'application en tant qu'inhibiteur de polymérisation, anti-oxydant dans les élastomères ou comme intermédiaire de synthèse. Un autre domaine d'application est la photographie. Il s'ensuit que c'est un produit de grande consommation.

Une pureté différente est requise selon les marchés concernés.

En effet, si certaines applications, en particulier la photographie requièrent un degré de pureté très élevée, d'autres se satisfont d'une hydroquinone technique c'est-à-dire ayant un degré de pureté moindre afin de minimiser les coûts d'exploitation.

Ainsi, l'hydroquinone doit satisfaire à des exigences de pureté variable et qui peuvent être dans certains cas, assez contraignantes.

Le problème qui se pose est que la purification n'est pas aisée car l'hydroquinone est un produit sensible à l'oxydation et qui conduit vite à des produits de dégradation qui sont colorés.

De plus, du pyrogallol est co-produit, certes en faibles quantités avec l'hydroquinone. Or, ce composé se dégrade très vite thermiquement et conduit à des impuretés colorées qu'il faut également éliminer.

Par ailleurs, des caractéristiques physico-chimiques différentes, en termes de granulométrie, coulabilité, vitesse de dissolution peuvent être requises selon les applications.

Donc, le marché est demandeur de produits diversifiés et possédant une bonne stabilité chimique au stockage.

Une des voies de synthèse de l'hydroquinone a été décrite dans FR 2.467.185. Elle consiste à produire simultanément du résorcinol et de l'hydroquinone par l'intermédaire d'un hydroperoxyde. Ce procédé conduit la purification de l'hydroquinone à partir d'un flux comportant une quantité élevée de résorcinol.

Une autre voie de synthèse de l'hydroquinone consiste à effectuer l'hydroxylation du phénol par le peroxyde d'hydrogène, notamment en présence de catalyseurs acides homogènes ou hétérogènes.

Ainsi, on peut faire appel comme selon FR 2 071 464, a un acide protique fort c'est-à-dire un acide présentant un pKa dans l'eau inférieur à 0,1, de préférence inférieur à -1.

Comme exemples d'acides protiques forts, on peut mentionner entre autres, l'acide sulfurique, l'acide chlorosulfurique, l'acide perchlorique, les acides sulfoniques comme par exemple, l'acide méthanesulfonique, trifluorométhanesulfonique, toluènesulfonique, phénolsulfonique.

Comme autres exemples de catalyseurs acides protiques, on peut citer les résines sulfoniques et plus particulièrement les résines commercialisées sous différentes dénominations commerciales. On peut citer, entre autres, les résines suivantes : TEMEX 50, AMBERLYST 15, AMBERLYST 35, AMBERLYST 36, DOWEX 50W.

Les résines précitées sont constituées d'un squelette polystyrénique qui porte des groupes fonctionnels qui sont des groupes sulfoniques. Le squelette polystyrénique est obtenu par polymérisation du styrène et du divinylbenzène, sous l'influence d'un catalyseur d'activation, le plus souvent un peroxyde organique, ce qui conduit à un polystyrène réticulé qui est ensuite traité par de l'acide sulfurique ou sulfochlorique concentré conduisant à un copolymère styrène-divinylbenzène sulfoné.

Il est également possible de faire appel à des résines sulfoniques qui sont des copolymères phénol-formol et qui portent sur le noyau aromatique un groupe méthylènesulfonique, par exemple la résine commercialisée sous la dénomination DUOLITE ARC 9359.

D'autres résines disponibles sur le marché conviennent également et l'on peut citer les résines perfluorées porteuses de groupes sulfoniques et, plus particulièrement le NAFION qui est un copolymère de tétrafluoroéthylène et de perfluoro[2-(fluorosulfonyléthoxy)-propyl] vinyl éther.

Comme autres catalyseurs convenant dans les procédés d'hydroxylation, on peut mentionner les complexes de fer II et de cuivre II (FR 2 121 000, USSR 1 502 559) et tout autre catalyseur de type Fenton.

D'autres procédés de préparation de l'hydroquinone font appel à une catalyse hétérogène. Ainsi, on peut mettre en oeuvre une zéolithe acide de type silicalite de titane (ou titanosilicalite-1) ou de fer de type TS-1 (FR 2 489 816), une zéolithe de type silicalite de titane MEL (EP 1 131 264) ou une titanozéosilite de type MFI (EP 1 123 159). Il est également possible d'utiliser une zéolithe MCM-22 (FR 2 856 681).

A l'issue de telles réactions d'hydroxylation, on obtient un mélange comprenant essentiellement du pyrocatéchol (ou 1,2-dihydroxybenzène) et de l'hydroquinone, en proportions variables avec en général, un ratio massique pyrocatéchol/ hydroquinone de l'ordre de 0,25 à 4,0, ainsi que différents sous-produits en des quantités beaucoup plus faibles, notamment du résorcinol (ou 1,3-dihydroxybenzène) et du pyrogallol (ou 1,2,3-trihydroxybenzène), généralement à des teneurs de l'ordre de 0,5 à 4,0 % en masse, pourcentages exprimés par rapport à la quantité d'hydroquinone et de pyrocatéchol formée.

On obtient des mélanges de compositions variables comprenant, en masse, de 20 à 80% de pyrocatéchol, de 80 à 20 % d'hydroquinone, de 0,1 à 2 % de résorcinol et de 0,1 à 2 % de pyrogallol.

Typiquement, on obtient des mélanges comprenant, en masse, de 50 à 80 % de pyrocatéchol, de 20 à 50 % d'hydroquinone, de 0,1 à 2 % de résorcinol et de 0,1 à 2 % de pyrogallol.

Pour isoler l'hydroquinone à partir de mélanges bruts de ce type, une méthode actuellement connue consiste à effectuer la distillation dudit mélange permettant d'obtenir en tête de distillation le pyrocatéchol (qui est le composé le plus volatil du mélange), et en pied de distillation, une "hydroquinone brute", à savoir un mélange contenant essentiellement de l'hydroquinone, associée à de faibles quantités d'impuretés (notamment résorcinol et pyrogallol ainsi que d'éventuelles traces de pyrocatéchol non éliminées par la distillation).

L'invention propose un procédé permettant à partir d'hydroquinone brute d'obtenir une hydroquinone ayant la pureté souhaitée.

Ainsi, l'objectif de l'invention est de proposer un procédé souple qui permet de contrôler la pureté de l'hydroquinone souhaitée et d'obtenir un produit qui puisse répondre à des exigences de pureté élevée.

Conformément au procédé de l'invention et selon le mode de réalisation choisi susceptible de comprendre un enchaînement différent des étapes, il est possible de moduler la pureté du produit obtenu.

Il a maintenant été trouvé et c'est ce qui constitue l'objet de la présente invention, un procédé de préparation d'hydroquinone purifiée à partir d'une hydroquinone brute *HQ⁰* contenant essentiellement de l'hydroquinone et de faibles quantités d'impuretés incluant au moins du résorcinol, du pyrogallol et des traces de pyrocatéchol caractérisé par le fait qu'il comprend au moins les étapes suivantes : la préparation d'un mélange comprenant en masse, de 20 à 80% de pyrocatéchol, de 80 à 20% d'hydroquinone, de 0,1 à 2% de résorcinol ; de 0,1 à 2% de pyrogallol, par hydroxylation du phénol par le peroxyde d'hydrogène en présence d'un catalyseur acide, la distillation dudit mélange afin d'obtenir le pyrocatéchol en tête et ladite hydroquinone brute en pied, la dissolution de ladite hydroquinone brute dans l'eau, la cristallisation de l'hydroquinone, la séparation de l'hydroquinone purifiée et une étape de séchage de l'hydroquinone purifiée.

Ainsi, selon l'invention, on obtient une hydroquinone dont la pureté est plus ou moins améliorée selon le type de modes de réalisation de l'invention.

La composition exacte de l'hydroquinone brute *HQ⁰* traitée selon les étapes du procédé de l'invention peut varier en une assez large mesure, le procédé de l'invention se révélant toutefois surtout intéressant pour des hydroquinones brutes comprenant de 96 à 99,5% masse d'hydroquinone et de faibles quantités d'impuretés inférieures à 10 % en masse (de préférence inférieures à 9 %). Les impuretés majoritaires sont le résorcinol et le pyrogallol: le pyrocatéchol représentant moins de 1 % de la masse d'impuretés. Le ratio pondéral pyrogallol/résorcinol varie généralement entre 0,2 et 5.

Le procédé de l'invention est particulièrement intéressant pour traiter des hydroquinones brutes comprenant de l'hydroquinone à raison de 96 à 99,5 % en masse, et des teneurs en impuretés de l'ordre de 0,5 à 4 %, par exemple de 0,5 à 2 % en masse, notamment de 1 à 2 % en masse par rapport à la masse totale de l'hydroquinone brute.

Typiquement, une hydroquinone brute *HQ⁰* traitée selon l'invention contient de 0,1 à 2 %, par exemple de 0,2 à 1 % en masse, d'impuretés légères constituées essentiellement par les isomères de l'hydroquinone à savoir le résorcinol et du pyrocatéchol résiduaire. Le résorcinol est en général une impureté majoritaire au sein des impuretés légères, les impuretés légères comprenant en règle générale au moins 50 % en masse de résorcinol par rapport à la masse totale des impuretés légères, par exemple au moins 70 %, notamment au moins 80 %, en particulier au moins 90 % en masse, voire plus. Outre le résorcinol, les impuretés légères présentes dans l'hydroquinone brute *HQ⁰* peuvent notamment comprendre du pyrocatéchol.

Par ailleurs, dans l'hydroquinone brute *HQ⁰*, la quantité d'impuretés lourdes dont la plus importante est constituée par le pyrogallol est usuellement de 0,1 à 2 %, par exemple de 0,2 à 1 % en masse. Ces impuretés lourdes incluent en particulier du pyrogallol, généralement à titre d'impureté lourde majoritaire, en général en association avec d'autres impuretés lourdes, notamment des goudrons ou bien encore des produits de dégradation thermique de l'hydroquinone tels que des quinones. Ainsi, les impuretés lourdes comprennent en général au moins 50 % en masse de pyrogallol par rapport à la masse totale des impuretés lourdes, par exemple au moins 70 %, voire au moins 80 %, en particulier au moins 90 % en masse ou plus.

Selon le procédé de l'invention, l'hydroquinone brute *HQ⁰* traitée selon le procédé de l'invention est obtenue à partir d'un mélange réactionnel issu d'une hydroxylation de phénol par le peroxyde d'hydrogène en présence de catalyseurs acides du type cité plus haut dans la présente description, après élimination substantielle du pyrocatéchol par distillation.

Une hydroquinone brute *HQ⁰* adaptée au procédé de l'invention comprend, en masse par rapport à la quantité totale d'hydroquinone brute :
- de 96 à 99,5 % d'hydroquinone,
- de 0,1 à 2 %, de préférence de 0,2 à 1 % de résorcinol,
- de 0,1 à 2 %, de préférence de 0,2 à 1 % de pyrogallol
- du pyrocatéchol sous forme de traces, typiquement à une teneur inférieure à 500 ppm (0,05 %), de préférence inférieure à 100 ppm (0,01 %).

Quelle que soit la nature exacte de l'hydroquinone brute *HQ⁰* traitée selon le procédé de l'invention, les étapes du procédé de l'invention sont avantageusement mises en oeuvre dans les conditions exposées ci-après.

Afin de faciliter la compréhension du procédé de l'invention, on donne ci-après, des figures de 1 à 4 qui schématisent les différentes variantes du procédé de l'invention, sans pour autant lier la portée de l'invention, à ceux-ci.

### Figure 1

Conformément au procédé de l'invention, on effectue d'abord la dissolution de l'hydroquinone dans l'eau, suivie par une cristallisation, une séparation solide/liquide et un séchage.

Plus précisément, le procédé comprend les étapes suivantes :
- dissolution de l'hydroquinone brute dans l'eau,
- cristallisation de l'hydroquinone,
- séparation solide/liquide permettant de séparer l'hydroquinone cristallisée d'une phase aqueuse constituée des eaux mères de cristallisation (*F₁*),
- séchage de l'hydroquinone lavée avec élimination d'eau (*F₂*).

On introduit l'hydroquinone et l'eau dans un réacteur agité dont la température peut être contrôlée soit à l'aide d'un échangeur thermique, soit par circulation d'un fluide caloporteur dans une double enveloppe dont il serait muni.

Comme fluides caloporteurs convenant à l'invention, on peut mentionner notamment l'eau ou bien un solvant organique tel que choisi parmi les esters lourds d'acides carboxyliques (par exemple, le phtalate d'octyle), les éthers aromatiques comme l'oxyde de biphényle et/ou l'oxyde de benzyle, le diphényle, les terphényles, les autres polyphényles éventuellement partiellement hydrogénés, les huiles paraffiniques et/ou naphténiques, résidus de distillation du pétrole etc...

La quantité d'eau introduite pour effectuer la dissolution de l'hydroquinone est telle que la concentration de l'hydroquinone dans l'eau est comprise entre 20 et 50 % en masse et de préférence entre 20 et 40 % en masse.

L'opération de dissolution est de préférence conduite à une température allant de 70°C à 100°C.

Les différents paramètres sont ajustables et la concentration peut être d'autant plus élevée que la température choisie est plus élevée.

Dans une étape suivante, on effectue la cristallisation de l'hydroquinone par refroidissement de la température de dissolution jusqu'à une température plus basse comprise entre 0 et 40°C, de préférence entre 5 à 25°C.

La cristallisation est effectuée dans les appareillages classiquement utilisés tels que dans des réacteurs agités (appelés cristallisoirs) avec échangeurs internes et/ou circulation d'un fluide caloporteur dans une double enveloppe. Le refroidissement peut également être effectué par évaporation partielle du solvant (eau) sous pression réduite (15 mBar et 250 mBar) et avec éventuellement recyclage des condensats.

L'opération dure généralement entre 60 et 720 min et est fonction du mode de cristallisation, de la taille des cristallisoirs et des charges ou débits d'alimentation.

On gère la taille des cristaux et le facteur d'élongation longueur sur largeur en jouant notamment sur les paramètres suivants : profil de température de refroidissement, puissance de l'agitation et temps de séjour dans le cristallisoir.

L'agitation est définie par la puissance dissipée qui est de préférence entre 0,4 et 1,2 kwatt/m³ de volume de réacteur.

Il est également possible de contrôler la granulométrie du produit obtenu en introduisant des germes de cristallisation à une teneur de préférence inférieure à 2 %. A titre de germes de cristallisation, on met en oeuvre une petite quantité d'hydroquinone cristallisée provenant d'une fabrication précédente et de granulométrie appropriée.

En fin d'opération, on obtient une suspension d'hydroquinone.

On effectue ensuite la séparation du produit cristallisé selon les techniques classiques de séparation solide/liquide, de préférence, par filtration, centrifugation ou essorage.

La séparation est généralement conduite à la température de fin de cristallisation, cependant une température différente peut être choisie.

On récupère un solide qui est essentiellement de l'hydroquinone purifée comprenant de 5 à 20 %, de préférence 5 et 10 % d'eau, selon la technique de séparation utilisée et une phase aqueuse constituée des eaux mères de cristallisation *(F₁)* comprenant essentiellement de l'eau, de l'hydroquinone à une teneur le plus souvent inférieure à environ 10%, de préférence inférieure à 7 % en masse.

Il est à noter que les opérations de dissolution et de cristallisation peuvent être réalisées en mode continu ou discontinu.

Selon une mise en oeuvre en continu, on peut prévoir une cascade de réacteurs et de cristallisoirs agités en série ou en parallèle avec des températures qui peuvent être différentes mais choisies dans la zone prédéfinie.

Comme mentionné précédemment, l'hydroquinone récupérée est soumise à une opération de séchage.

La température de séchage est avantageusement choisie entre 100°C et 140°C.

Le séchage est conduit selon les techniques bien connues de l'Homme du Métier et dans les appareillages classiquement utilisés tels que les séchoirs par contact à pression atmosphérique ou sous pression réduite, les séchoirs convectifs par l'air (type séchoir pneumatique) ou un gaz inerte de préférence l'azote avec possibilité de recyclage du gaz inerte. On peut également sécher l'hydroquinone selon la technique du lit fluidisé.

Suite au séchage, on obtient un flux *(F₂)* constitué essentiellement d'eau et le produit séché à savoir l'hydroquinone *HQ¹* dont les caractéristiques sont les suivantes :
- la teneur en hydroquinone est supérieure ou égale à 98,5 %,
- la teneur en résorcinol est inférieure à 4000 ppm,
- la teneur en pyrogallol est inférieure à 4000 ppm,
- la coloration est inférieure à 500 Hazen.

La coloration est déterminée à température ambiante par spectrocolorimétrie sur une solution aqueuse d'hydroquinone à 5 % en masse dans l'eau distillée.

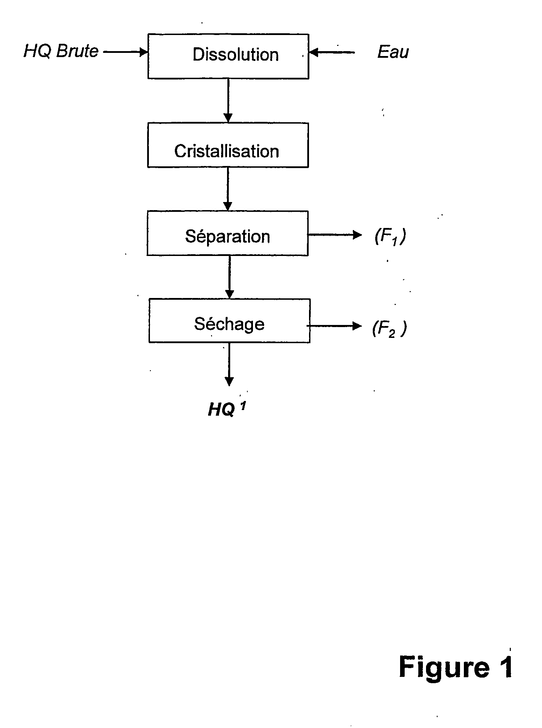

Selon le mode de réalisation de la figure 1, la teneur obtenue en hydroquinone est inférieure ou égale à 99,2 % environ.

### Figure 2.

Conformément au procédé de l'invention, on obtient une hydroquinone plus pure par rapport à *HQ²* en effectuant une étape additionnelle.

Ainsi, le procédé de l'invention, selon ce mode de réalisation comprend la dissolution de l'hydroquinone dans l'eau, suivie par une cristallisation, une séparation solide/liquide, un lavage et un séchage.

Plus précisément, le procédé comprend les étapes suivantes :
- dissolution de l'hydroquinone brute dans l'eau,
- cristallisation de l'hydroquinone,
- séparation solide/liquide permettant de séparer l'hydroquinone cristallisée d'une phase aqueuse constituée des eaux mères de cristallisation *(F₁)*,
- lavage de l'hydroquinone séparée permettant de recueillir des eaux de lavage *(F₃)*,
- séchage de l'hydroquinone lavée avec élimination d'eau *(F₄)*.

On effectue la dissolution de l'hydroquinone dans l'eau, la cristallisation et la séparation comme décrit ci-dessus mais l'on réalise ensuite le lavage de l'hydroquinone séparée avant un éventuel séchage.

Le lavage est conduit en mettant en oeuvre une quantité d'eau allant de 20 à 60 % de la masse d'hydroquinone. Le lavage peut être fait en une ou plusieurs fois.

Le lavage peut être réalisé sur le même appareillage de séparation solide/liquide ; soit sur filtré, sur essoreuse ou dans la centrifugeuse ou bien selon une opération de repulpage qui consiste à récupérer l'hydroquinone et l'introduire dans un autre réacteur avec de l'eau puis à séparer l'hydroquinone lavée.

En fin d'opération, on recueille les eaux de lavage *(F₃)* comprenant majoritairement de l'eau et minoritairement (moins de 10 % en masse) de l'hydroquinone et des impuretés.

L'hydroquinone lavée est soumise à une opération de séchage comme décrite précédemment.

Suite au séchage, on obtient un flux *(F₄)* constitué essentiellement d'eau et le produit séché à savoir l'hydroquinone *HQ²* dont les caractéristiques sont les suivantes :
- la teneur en hydroquinone est supérieure ou égale à 99,5 %,
- la teneur en résorcinol est inférieure à 500 ppm,
- la teneur en pyrogallol est inférieure à 100 ppm,
- la coloration est inférieure à 30 Hazen.

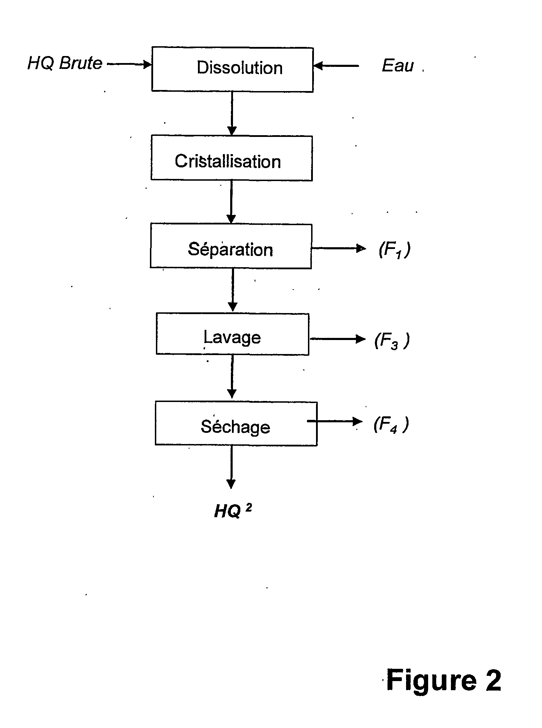

Selon le mode de réalisation de la figure 2, la teneur obtenue en hydroquinone est inférieure ou égale à 99,9 % environ.

### Figure 3.

Conformément au procédé de l'invention, on obtient une hydroquinone *HQ³* de qualité différente selon un procédé comprenant les étapes suivantes :
- dissolution de l'hydroquinone brute dans l'eau,
- cristallisation de l'hydroquinone,
- séparation solide/liquide permettant de séparer l'hydroquinone cristallisée d'une phase aqueuse constituée des eaux mères de cristallisation *(F₁ₐ)*,
- lavage de l'hydroquinone séparée permettant de recueillir des eaux de lavage *(F₃ₐ)*,
- séchage de l'hydroquinone lavée avec élimination d'eau *(F₄ₐ)*,
- mélangeage des flux *(F₁ₐ)* et *(F₃ₐ)* récupérés,
- concentration du mélange obtenu,
- et recyclage de celui-ci à l'étape de dissolution ou de cristallisation.

Selon cette variante du procédé de l'invention, on répète les étapes décrites précédemment et l'on mélange la phase aqueuse *(F₁ₐ)* obtenue suite à la séparation avec les eaux de lavage récupérées *(F₃ₐ)*.

Cette opération est effectuée dans un réacteur agité quelconque.

Le mélangeage est conduit à une température choisie avantageusement entre 5°C et 70°C.

Selon une caractéristique du procédé de l'invention, on peut effectuer une concentration du mélange obtenu de façon à augmenter la concentration en hydroquinone dans le milieu de 5 % en masse à 40 % en masse, de préférence de 7 % à 35 %.

Ainsi, on élimine au cours de cette opération, un flux *(F₅ₐ)* comprenant de l'eau (par exemple de 70 à 90 % poids par rapport à la masse du milieu réactionnel issu de l'opération de mélangeage).

Un premier mode consiste tout en restant dans la zone de température précitée, à diminuer la pression réactionnelle, par détente. Cette détente est réalisée de façon à éliminer en tête la quantité d'eau nécessaire pour atteindre dans le milieu réactionnel la concentration cible en hydroquinone.

Par élimination d'eau et d'impuretés volatiles, on obtient la concentration souhaitée en hydroquinone.

Un autre mode de réalisation pour concentrer le milieu réactionnel consiste à effectuer la distillation de la quantité d'une partie de l'eau pour atteindre dans le milieu réactionnel, la concentration souhaitée en hydroquinone.

On peut effectuer la distillation sous pression atmosphérique à une température de l'ordre de 100°C.

On peut également effectuer la distillation sous une pression légèrement inférieure à la pression atmosphérique, par exemple de 200 à 750 mm de mercure (soit de 2,66×10⁴ à 9,99×10⁴ Pa), et à une température inférieure à 100°C. En général, la pression est choisie pour avoir une température de distillation située entre 70°C et 100°C.

On peut aussi effectuer la distillation sous pression supérieure à la pression atmosphérique.

Ces opérations sont conduites dans des appareillages classiques tels que colonnes à distiller ou évaporateurs standards sous pression.

Ce flux concentré est avantageusement recyclé soit à l'étape de dissolution, soit à l'alimentation de cristallisation.

Afin de minimiser les impuretés présentes dans cette boucle de recirculation, il est préférable d'effectuer une purge ou plusieurs purges d'un des flux comprenant les impuretés.

La purge consiste à enlever une fraction dudit flux. Elle est déterminée de telle sorte que la perte globale d'hydroquinone par rapport à l'hydroquinone brute soit comprise préférentiellement entre 0,5 et 2 % en masse.

Elle peut être faite en (P₁), de préférence en sortie de la zone de concentration. Elle est exécutée par l'intermédiaire d'une vanne placée sur le conduit véhiculant le flux concentré, en sortie de l'opération de concentration mais avant recyclage.

Il est également possible de faire une purge en (P₂) sur le flux constitué par la phase aqueuse *(F₁ₐ)* issue de la séparation de l'hydroquinone après cristallisation mais avant mélangeage avec le flux *(F₃ₐ)* constitué par les eaux de lavage.

Il est préférable d'effectuer la purge en sortie de la zone de concentration.

La purge peut être faite en continu ou en discontinu

La maîtrise de la purge permet de régler la pureté chimique de l'hydroquinone obtenue ainsi que le rendement global du procédé.

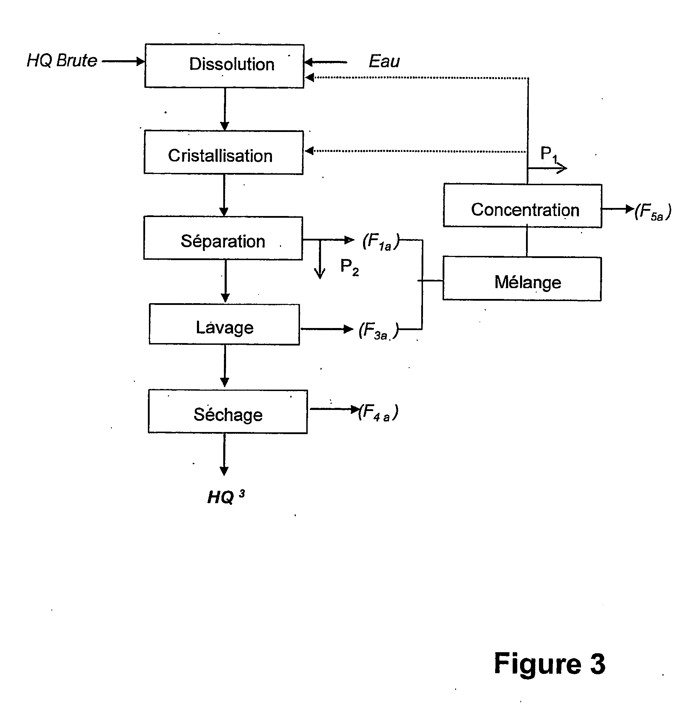

Suite aux différentes opérations définies selon le schéma de la figure 3, on obtient de l'hydroquinone *HQ³* dont les caractéristiques sont les suivantes :
- la teneur en hydroquinone est supérieure ou égale à 99 %,
- la teneur en résorcinol est inférieure à 5000 ppm,
- la teneur en pyrogallol est inférieure à 200 ppm,
- la coloration est inférieure à 50 Hazen.

Selon le mode de réalisation de la figure 3, la teneur obtenue en hydroquinone est inférieure ou égale à 99,5 % environ.

### Figure 4.

Selon un autre mode de réalisation de l'invention, on récupère de l'hydroquinone dite « technique » à partir des flux *(F₁)* et *(F₃)* issus des opérations de séparation et de lavage comme décrit dans la figure 2.

Pour ce faire, on met en oeuvre un procédé comprenant les étapes suivantes :
- mélangeage des flux *(F₁)* et *(F₃)* récupérés avec de l'hydroquinone brute,
- concentration du mélange obtenu conduisant à l'élimination d'un flux *(F₅)*,
- cristallisation de l'hydroquinone présente dans le flux concentré,
- séparation solide/liquide permettant de séparer l'hydroquinone cristallisée d'une phase aqueuse constituée des eaux mères de cristallisation *(F₆)*,
- lavage de l'hydroquinone séparée permettant de recueillir des eaux de lavage *(F₇)*,
- séchage de l'hydroquinone lavée avec élimination d'eau *(F₈),*
- mélangeage des flux *(F₆)* et *(F₇)* récupérés,
- et recyclage du mélange obtenu à l'étape de mélangeage des flux *(F₁)* et *(F₃)*.

Selon cette variante de réalisation, on mélange les flux *(F₁)* et *(F₃)* récupérés avec de l'hydroquinone brute. La quantité d'hydroquinone recyclée *(F₁)* + *(F₃)* est adaptée selon la qualité de l'hydroquinone souhaitée. Généralement, elle représente de 40 à 80 % de la masse du mélange obtenu.

Comme précédemment décrit dans le schéma de la figure 3, on effectue une purge en (P₃) de la phase aqueuse *(F₆)* issue de la séparation de l'hydroquinone récupérée à partir du flux concentré avant mélangeage avec le flux *(F₇)* constitué par les eaux de lavage.

Le mélangeage des flux *(F₆)* et *(F₇)* récupérés est généralement effectué à une température allant de 5°C à 70°C.

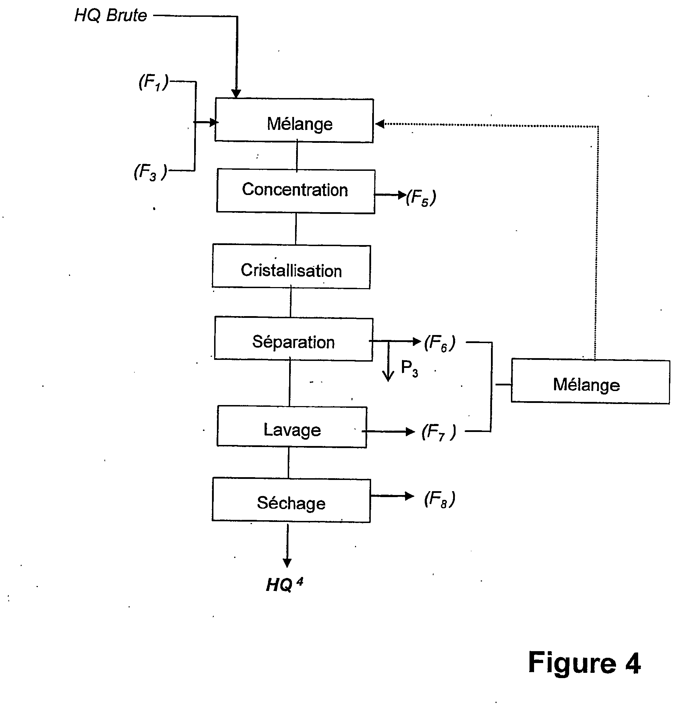

Suite aux différentes opérations définies selon le schéma de la figure 4, on obtient de l'hydroquinone *HQ⁴* dont les caractéristiques sont les suivantes :
- la teneur en hydroquinone est supérieure ou égale à 99 %,
- la teneur en résorcinol est inférieure à 5000 ppm,
- la teneur en pyrogallol est inférieure à 500 ppm,
- la coloration est inférieure à 100 Hazen.

Selon le mode de réalisation de la figure 4, la teneur obtenue en hydroquinone est inférieure ou égale à 99,4 % environ.

Conformément au procédé de l'invention, on purifie l'hydroquinone obtenue à partir d'un mélange réactionnel issu d'une hydroxylation de phénol par le peroxyde d'hydrogène en présence d'un catalyseur acide, après élimination substantielle du pyrocatéchol par distillation, en mettant en oeuvre une suite d'étapes choisies en fonction de l'objectif visé.

D'une manière avantageuse, les différentes opérations précédemment citées, dissolution, cristallisation, séparation, séchage etc... sont conduites sous atmosphère de gaz inertes, de préférence sous azote.

Il est à noter que l'invention n'exclut pas des étapes additionnelles insérées dans les enchaînements définis par l'invention en particulier l'ajout de traitement usuel des solides en particulier un traitement au noir de carbone (ou charbon actif), en vue d'améliorer la blancheur du produit obtenu.

Par exemple, selon les schémas des figures 1 à 3, il est possible de conduire ce traitement avant l'opération de cristallisation et suite à l'opération de dissolution, en ajoutant une quantité de noir de carbone ou de charbon actif à raison de 0,02 à 0,50 % par rapport à la masse d'hydroquinone.

Ce traitement est effectué dans un réacteur agité.

Il est suivi d'une séparation solide/liquide, de préférence une filtration pour éliminer le noir de carbone suivi avantageusement par une opération de lavage.

Un autre mode de réalisation du traitement au noir est d'utiliser la technique du lit fixe.

Ainsi le flux issu de l'opération de dissolution est envoyé sur un lit fixe de noir de carbone de préférence sous forme de granulés.

Les granulés de noir de carbone sont placés dans une colonne et le flux contenant l'hydroquinone passe généralement à co-courant au travers du lit fixe.

En ce qui concerne le schéma de la figure 4, il est également possible d'effectuer un traitement au noir comme décrit précédemment, avant la cristallisation.

Ainsi, le procédé de l'invention est particulièrement intéressant car il permet d'obtenir l'hydroquinone à différents degrés de pureté s'échelonnant entre au moins 98% et pouvant être proche de 100 %, par exemple égal à 99,9 %.

Les bornes inférieures des puretés atteintes par le procédé de l'invention sont aux environ de 50 ppm pour le résorcinol, 50 ppm pour le pyrogallol et 20 ppm pour le pyrocatéchol.

Enfin, il est à souligner que le procédé de l'invention est intéressant d'un point de vue économique car le rendement de purification reste toujours supérieur à 95 % en choisissant le mode de réalisation adéquate parmi ceux proposés par la présente invention.

Il est à noter que l'hydroquinone purifiée obtenue selon le procédé de l'invention, a un aspect blanc. La coloration de l'hydroquinone est mesurée comme précisée ci-dessus par spectrocolorimétrie. On obtient toujours pour l'hydroquinone obtenue selon l'invention, un indice de colorimétrie inférieur ou égal à 500 Hazen et généralement supérieur ou égal à 5 Hazen et compris le plus souvent entre 10 et 100 Hazen.

On précise que les différentes pourcentages pondéraux donnés dans le présent texte, en rapport à l'hydroquinone sous forme solide, sont exprimés par rapport à un produit sec obtenu après séchage jusqu'à obtention d'un poids constant.

On donne ci-après des exemples de réalisation de l'invention donnés à titre illustratif et sans caractère limitatif.

Les exemples 1 à 4 se réfèrent respectivement aux figures 1 à 4.

### EXEMPLES

### Exemple 1

Dans un cristallisoir agité à double enveloppe de 5 litres équipé d'un condenseur, maintenu sous azote et à une température de 95°C, on coule respectivement 2,234 kg d'eau à température ambiante et 1,266 kg d'hydroquinone brute dont la composition en % masse est la suivante : 97,8 % d'hydroquinone, 1,1 % de pyrogallol et 1,1 % de résorcinol ; la somme des autres impuretés présentes étant inférieure à 0,1 %.

Un refroidissement rapide en 30 min jusqu'à 70°C est réalisé.

Un refroidissement à vitesse constante de 70°C à 20°C, en 5 heures est alors réalisé.

La suspension d'hydroquinone cristallisée est alors filtrée sur filtre plan sous azote pour obtenir 1,369 kg d'hydroquinone humide et 2,131 kg de jus mères (F₁).

La solution de jus mères (F₁) est conservée pour traitement ultérieur.

On sèche l'hydroquinone humide, en étuve sous pression réduite (100 mBar) et sous balayage d'azote, à 90°C, pendant 5 heures avec élimination de 0,254 kg d'eau (F₂).

On isole 1,115 kg d'hydroquinone *HQ¹* de composition en masse suivante :
- Teneur en hydroquinone = 99 %
- Teneur en pyrogallol : 2300 ppm
- Teneur en résorcinol : 2500 ppm
- Teneur en pyrocatéchol : 30 ppm
- Teneur en eau 0,5 %
- Colocation : 350 Hazen

### Exemple 2

Dans un cristallisoir agité à double enveloppe de 5 litres équipé d'un condenseur, maintenu sous azote et à une température de 95°C, on coule respectivement 2,346 kg d'eau à température ambiante et 1,330 kg d'hydroquinone brute dont la composition en % masse est la suivante : 97,2 %, d'hydroquinone, 1,4 % de pyrogallol et 1,4 % de résorcinol ; la somme des autres impuretés présentes étant inférieure à 0,2 %.

Un refroidissement rapide en 30 min jusqu'à 70°C est réalisé.

Un refroidissement à vitesse constante de 70°C à 20°C en 5 heures est alors réalisé.

La suspension d'hydroquinone cristallisée est alors filtrée sur filtre plan sous azote pour obtenir 1,565 kg d'hydroquinone humide et 2,111kg de jus mères (F₁) conservés pour traitement ultérieur.

Un lavage à température de 20°C avec 0,500 kg d'eau est réalisé sur le filtre.

On récupère séparément, 0,525 kg de jus de lavages (F₃) pour un traitement ultérieur.

On sèche 1,540 kg du gâteau lavé, en étuve sous pression réduite et sous balayage d'azote à 90°C, pendant 5 heures et l'on évapore 0,247 kg d'eau (F₄).

On isole 1,293 kg d'hydroquinone *HQ²* de composition en masse suivante :
- Teneur en hydroquinone = 99,5 %
- Teneur en pyrogallol : 70 ppm
- Teneur en résorcinol : 250 ppm
- Teneur en pyrocatéchol : 20 ppm
- Teneur en eau : 0,40 %
- Coloration : 20 Hazen

### Exemple 3

Dans un cristallisoir agité à double enveloppe de 5 litres équipé d'un condenseur, maintenu sous azote et à une température de 95°C, on coule respectivement 1,689 kg de jus mères (F₁) de l'exemple 2, 0,525 kg de jus de lavages (F₃) de l'exemple 2 et 0,823 kg d'eau à température ambiante et 1,330 kg d'hydroquinone brute dont la composition en % masse est la suivante : 97,2 % d'hydroquinone 1,4 % de pyrogallol et 1,4 % de résorcinol ; la somme des autres impuretés présentes étant inférieure à 0,2 %.

Un refroidissement rapide en 30 min jusqu'à 70°C est réalisé.

Un refroidissement à vitesse constante de 70°C à 20°C en 10 heures est alors réalisé.

La suspension d'hydroquinone cristallisée est alors filtrée sur filtre plan sous azote pour obtenir 1,527 kg d'hydroquinone humide et 2,840 kg de jus mères (F₁ₐ).

Un lavage à température de 20°C, avec 0,500 kg d'eau, est réalisé sur le filtre.

On récupère séparément, 0,535 kg de jus de lavages (F₃ₐ).

On sèche 1,493 kg de ce gâteau en étuve, sous pression réduite et sous balayage d'azote à 90°C, pendant 5 heures et l'on évapore 0,210 kg d'eau (F₄ₐ).

On isole 1,283 kg d'hydroquinone *HQ³* de composition en masse suivante :
- Teneur en HQ = 99,0 %
- Teneur en pyrogallol: 110 ppm
- Teneur en résorcinol : 1600 ppm
- Teneur en pyrocatéchol : 25 ppm
- Teneur en eau : 0,40 %
- Coloration : 35 Hazen

### Exemple 4

Dans un cristallisoir agité à double enveloppe de 5 litres équipé d'un condenseur, maintenu sous azote et à une température de 95°C, on coule respectivement 2,346 kg d'eau à température ambiante et 1,330 kg d'hydroquinone brute dont la composition en % masse est la suivante : 97,2 % d'hydroquinone, 1,4 % de pyrogallol et 1,4 % de résorcinol ; la somme des autres impuretés présentes étant inférieure à 0,2 %.

Un refroidissement rapide en 30 min jusqu'à 70°C est réalisé.

Un refroidissement à vitesse constante de 70°C à 20°C en 5 heures est alors réalisé.

La suspension d'hydroquinone cristallisée est alors filtrée sur filtre plan sous azote pour obtenir 1,565 kg d'hydroquinone humide et 2,111kg de jus mères (F₁).

Un lavage à température de 20°C, avec 0,500 kg d'eau est réalisé sur le filtre.

On récupère séparément 0,525 kg de jus de lavages (F₃).

On reproduit à l'identique cette cristallisation à 4 reprises.

On mélange l'ensemble des flux (F₁) et (F₃) dans un évaporateur rotatif pour effectuer la concentration par distillation à pression atmosphérique et obtenir 2,237 kg de solution concentrée d'hydroquinone à 33 % en masse après évaporation de 8,307 kg d'eau (F₅).

Dans un cristallisoir agité à double enveloppe de 3 litres, équipé d'un condenseur, maintenu sous azote et à une température de 90°C, on charge les 2,237 kg précédents.

Un refroidissement rapide en 20 min jusqu'à 70°C est réalisé.

Un refroidissement à vitesse constante de 70 à 20°C en 12 heures est alors réalisé.

La suspension d'hydroquinone cristallisée est alors filtrée sur filtre plan sous azote pour obtenir 0,710 kg d'hydroquinone humide et 1,527 kg de jus mères (F₆).

Un lavage à une température de 20°C avec 0,300 kg d'eau, est réalisé sur le filtre.

On récupère séparément 0,315 kg de jus de lavages (F₇).

On réalise ensuite l'enchaînement décrit ci-après.

Dans un cristallisoir agité à double enveloppe de 5 litres équipé d'un condenseur, maintenu sous azote et à une température de 95°C, on coule respectivement 1,527 Kg de jus mères précédents (F₆), 0,315 kg de jus de lavages précédents (F₇), et 0,665 kg d'hydroquinone brute dont la composition en % masse est la suivante : 97,2 % d'hydroquinone, 1,4 % de pyrogallol et 1,4 % de résorcinol ; la somme des autres impuretés présentes étant inférieure à 0,2 %.

Dans cet exemple, la totalité des jus mères (F₆) est utilisée. (Cependant il est intéressant pour éviter de dégrader la qualité de n'utiliser qu'une partie des jus mères.)

La nouvelle composition a une teneur en hydroquinone d'environ 33 % en masse.

Un refroidissement rapide en 30 min jusqu'à 70°C est réalisé.

Un refroidissement à vitesse constante de 70°C à 20°C en 12 heures est alors réalisé.

La suspension d'hydroquinone cristallisée est alors filtrée sur filtre plan sous azote pour obtenir 0,810 kg d'hydroquinone humide et 1,697 kg de jus mères (F₆).

Un lavage à température de 20°C, avec 0,750 kg d'eau, est réalisé sur le filtre.

On récupère séparément 0,805 kg de jus de lavages (F₇) et un gâteau humide d'hydroquinone de 0,755 kg.

On sèche 0,755 kg de ce gâteau en étuve sous pression réduite et sous balayage d'azote à 90°C, pendant 8 heures et l'on évapore 0,110 kg d'eau (F₈) d'eau.

On isole 0,645 kg d'hydroquinone *HQ⁴* de composition en masse suivante :
- Teneur en HQ = 99,0 %
- Teneur en pyrogallol : 220 ppm
- Teneur en résorcinol : 1500 ppm
- Teneur en pyrocatéchol : 25 ppm
- Teneur en eau : 0,32 %
- Coloration : 60 Hazen

Il doit être bien compris que l'invention définie par les revendications annexées n'est pas limitée aux modes de réalisation particuliers indiqués dans la description ci-dessus.

Ainsi, l'invention englobe les modes de réalisation qui résulteraient de la combinaison des schémas définis selon les figures 1 à 4.

## Revendications

1. - Procédé de préparation d'hydroquinone purifiée à partir d'une hydroquinone brute *HQ⁰* comprenant de 96 à 99,5 % masse d'hydroquinone et de faibles quantités d'impuretés incluant de 0,1 à 2% masse de résorcinol, de 0,1 à 2% masse de pyrogallol (les pourcentages étant exprimés par rapport à la quantités totale d'hydroquinone brute) et des traces de pyrocatéchol **caractérisé par le fait qu'**il comprend au moins les étapes suivantes :
- la préparation d'un mélange comprenant, en masse, de 20 à 80% de pyrocatéchol, de 80 à 20% d'hydroquinone, de 0,1 à 2% de résorcinol, de 0,1 à 2% de pyrogallol, par hydroxylation du phénol par le peroxyde d'hydrogène en présence d'un catalyseur acide,
- la distillation dudit mélange afin d'obtenir le pyrocatéchol en tête et ladite hydroquinone brute en pied,
- la dissolution de ladite hydroquinone brute dans l'eau,
- la cristallisation de l'hydroquinone
- la séparation de l'hydroquinone purifiée
- et une étape de séchage de l'hydroquinone purifiée.

2. - Procédé selon la revendication 1 **caractérisé par le fait qu'**il comprend une étape de lavage avant l'étape de séchage.

3. - Procédé selon l'une des revendications 1 à 2 **caractérisé par le fait qu'**il comprend les étapes suivantes :
- dissolution de l'hydroquinone brute dans l'eau,
- cristallisation de l'hydroquinone,
- séparation solide/liquide permettant de séparer l'hydroquinone cristallisée d'une phase aqueuse constituée des eaux mères de cristallisation *(F₁ₐ)*,
- lavage de l'hydroquinone séparée permettant de recueillir des eaux de lavage *(F₃ₐ)*,
- séchage de l'hydroquinone lavée avec élimination d'eau *(F₄ₐ)*,
- mélangeage des flux *(F₁ₐ)* et *(F₃ₐ)* récupérés,
- concentration du mélange obtenu,
- et recyclage de celui-ci à l'étape de dissolution ou de cristallisation.

4. - Procédé selon la revendication 3 **caractérisé par le fait que** l'on effectue une purge en (P₁), de préférence en sortie de la zone de concentration ou en (P₂), sur le flux constituée par la phase aqueuse *(F₁ₐ)* issue de la séparation de l'hydroquinone après cristallisation mais avant mélangeage avec le flux *(F₃ₐ)* constitué par les eaux de lavage.

5. - Procédé selon l'une des revendications 1 à 2 **caractérisé par le fait qu'**il comprend les étapes suivantes :
- mélangeage des flux *(F₁)* et *(F₃)* récupérés avec de l'hydroquinone brute,
- concentration du mélange obtenu conduisant à l'élimination d'un flux *(F₅),*
- cristallisation de l'hydroquinone présente dans le flux concentré,
- séparation solide/liquide permettant de séparer l'hydroquinone cristallisée d'une phase aqueuse constituée des eaux mères de cristallisation *(F₆),*
- lavage de l'hydroquinone séparée permettant de recueillir des eaux de lavage *(F₇),*
- séchage de l'hydroquinone lavée avec élimination d'eau *(F₈),*
- mélangeage des flux *(F₆)* et *(F₇)* récupérés,
- et recyclage du mélange obtenu à l'étape de mélangeage des flux *(F₁)* et *(F₃).*

6. - Procédé selon la revendication 5 **caractérisé par le fait que** l'on effectue une purge en (P₃) de la phase aqueuse *(F₆)* issue de la séparation de l'hydroquinone récupérée à partir du flux concentré, avant mélangeage avec le flux *(F₇)* constitué par les eaux de lavage.

7. - Procédé selon l'une des revendications 1 à 6 **caractérisé par le fait que** la dissolution est faite par introduction d'une quantité d'eau telle que la concentration de l'hydroquinone dans l'eau est comprise entre 20 et 50 % en masse, de préférence entre 20 et 40 % en masse.

8. - Procédé selon la revendication 7 **caractérisé par le fait que** l'opération de dissolution est de préférence conduite à une température allant de 70°C à 100°C.

9. - Procédé selon l'une des revendications 1 à 8 **caractérisé par le fait que** l'on effectue la cristallisation de l'hydroquinone par refroidissement de la température de dissolution jusqu'à une température plus basse comprise entre 0°C et 40°C, de préférence entre 5°C et 25°C.

10. - Procédé selon l'une des revendications 1 à 9 **caractérisé par le fait que** la température de séchage est avantageusement choisie entre 100°C et 140°C.

11. - Procédé selon l'une des revendications 1 à 10 **caractérisé par le fait que** le lavage est conduit en mettant en oeuvre une quantité d'eau allant de 20 à 60 % de la masse d'hydroquinone.

12. - Procédé selon la revendication 3 **caractérisé par le fait que** le mélangeage des flux *(F₁ₐ)* et *(F₃ₐ)* est conduit à une température choisie avantageusement entre 5°C et 70°C.

13. - Procédé selon l'une des revendications 1 et 12 **caractérisé par le fait que** l'on effectue une concentration du mélange obtenu de façon à augmenter la concentration en hydroquinone dans le milieu de 5 % en masse à 40% en masse, de préférence de 7 % à 35 %.

14. - Procédé selon la revendication 13 **caractérisé par le fait que** l'on effectue la concentration par diminution de la pression tout en restant dans la zone de température précitée ; ou par distillation sous pression atmosphérique ou à des pressions inférieures ou supérieures à la pression atmosphérique.

15. - Procédé selon la revendication 5 **caractérisé par le fait que** le mélangeage des flux *(F₆)* et *(F₇)* est conduit à une température choisie avantageusement entre 5°C et 70°C.

16. - Procédé selon l'une des revendications 1 à 15 **caractérisé par le fait que** l'hydroquinone brute *HQ⁰* traitée a des teneurs en impuretés de 0,5 à 4%, de préférence de 0,5 à 2% en masse, et encore plus préférentiellement de 1 à 2% en masse par rapport à la masse totale de l'hydroquinone brute.

17. - Procédé selon l'une des revendications 1 à 16 **caractérisé par le fait que** l'hydroquinone brute *HQ⁰* traitée contient de 0,1 à 2 %, de préférence de 0,2 à 1 % en masse, d'impuretés légères constituées essentiellement du résorcinol et du pyrocatéchol résiduaire.

18. - Procédé selon l'une des revendications 1 à 17 **caractérisé par le fait que** l'hydroquinone brute *HQ⁰* traitée contient de 0,1 à 2 %, de préférence de 0,2 à 1 % en masse de pyrogallol.

19. - Procédé selon l'une des revendications 1 à 18 **caractérisé par le fait que** l'hydroquinone brute *HQ⁰* comprend, en masse par rapport à la quantité totale d'hydroquinone brute :
- de 96 à 99,5 % d'hydroquinone,
- de 0,1 à 2 % de résorcinol,
- de 0,1 à 2 % de pyrogallol
- du pyrocatéchol sous forme de traces à une teneur inférieure à 500 ppm (0,05 %).

20. - Procédé selon l'une des revendications 1 à 19 **caractérisé par le fait que** le catalyseur acide est choisi parmi :
- les acides protiques forts, de préférence l'acide sulfurique, l'acide chlorosulfurique, l'acide perchlorique, les acides sulfoniques de préférence, l'acide méthanesulfonique, trifluorométhanesulfonique, toluènesulfonique, phénolsulfonique,
- les résines sulfoniques, de préférence un copolymère styrène-divinylbenzène sulfoné, un copolymère phénol-formol qui porte sur le noyau aromatique un groupe méthylènesulfonique, une résine perfluorée porteuse de groupes sulfoniques,
- les complexes de fer II et de cuivre II,
- les zéolithes acides, de préférence les zéolithes de type silicalite de titane (ou titanosilicalite-1) ou de fer de type TS-1, les zéolithes de type silicalite de titane MEL ou les titanozéosilites de type MFI, les zéolithes MCM-22.

21. - Procédé selon la revendication 1 **caractérisé par le fait que** l'on obtient de l'hydroquinone *HQ¹* dont les caractéristiques sont les suivantes :
- la teneur en hydroquinone est supérieure ou égale à 98,5 %,
- la teneur en résorcinol est inférieure à 4000 ppm,
- la teneur en pyrogallol est inférieure à 4000 ppm,
- la coloration est inférieure à 500 Hazen.

22. - Procédé selon la revendication 2 **caractérisé par le fait que** l'on obtient de l'hydroquinone *HQ²* dont les caractéristiques sont les suivantes :
- la teneur en hydroquinone est supérieure ou égale à 99,5 %,
- la teneur en résorcinol est inférieure à 500 ppm,
- la teneur en pyrogallol est inférieure à 100 ppm,
- la coloration est inférieure à 30 Hazen.

23. - Procédé selon la revendication 3 **caractérisé par le fait que** l'on obtient de l'hydroquinone *HQ³* dont les caractéristiques sont les suivantes :
- la teneur en hydroquinone est supérieure ou égale à 99 %,
- la teneur en résorcinol est inférieure à 5000 ppm,
- la teneur en pyrogallol est inférieure à 200 ppm,
- la coloration est inférieure à 50 Hazen.

24. - Procédé selon la revendication 5 **caractérisé par le fait que** l'on obtient de l'hydroquinone *HQ⁴* dont les caractéristiques sont les suivantes :
- la teneur en hydroquinone est supérieure ou égale à 99 %,
- la teneur en résorcinol est inférieure à 5000 ppm,
- la teneur en pyrogallol est inférieure à 500 ppm,
- la coloration est inférieure à 100 Hazen.

## Claims

1. Process for the preparation of purified hydroquinone starting from a crude hydroquinone *HQ⁰* comprising from 96 to 99.5% by weight of hydroquinone and low amounts of impurities, including from 0.1 to 2% by weight of resorcinol, from 0.1 to 2% by weight of pyrogallol (the percentages being expressed with respect to the total amount of crude hydroquinone) and traces of pyrocatechol, **characterized in that** it comprises at least the following stages:
- the preparation of a mixture comprising, by weight, from 20 to 80% of pyrocatechol, from 80 to 20% of hydroquinone, from 0.1 to 2% of resorcinol and from 0.1 to 2% of pyrogallol, by hydroxylation of phenol by hydrogen peroxide in the presence of an acid catalyst,
- the distillation of said mixture in order to obtain pyrocatechol at the top and the said crude hydroquinone at the bottom,
- the dissolution of said crude hydroquinone in water,
- the crystallization of the hydroquinone,
- the separation of the purified hydroquinone
- and a stage of drying the purified hydroquinone.

2. Process according to Claim 1, **characterized in that** it comprises a washing stage before the drying stage.

3. Process according to either of Claims 1 and 2, **characterized in that** it comprises the following stages:
- dissolution of the crude hydroquinone in water,
- crystallization of hydroquinone,
- solid/liquid separation, making it possible to separate the crystalline hydroquinone from an aqueous phase composed of the crystallization aqueous mother liquors *(F₁ₐ),*
- washing the separated hydroquinone, making it possible to collect aqueous wash liquors *(F₃ₐ)*,
- drying the washed hydroquinone with removal of water *(F₄ₐ),*
- mixing the recovered streams *(F₁ₐ)* and *(F₃ₐ),*
- concentrating the mixture obtained,
- and recycling the latter to the dissolution or crystallization stage.

4. Process according to Claim 3, **characterized in that** a bleeding is carried out at (P₁), preferably at the outlet of the concentrating region, or at (P₂), on the stream composed of the aqueous phase *(F₁ₐ)* resulting from the separation of the hydroquinone after crystallization but before mixing with the stream *(F₃ₐ)* composed of the aqueous wash liquors.

5. Process according to either of Claims 1 and 2, **characterized in that** it comprises the following stages:
- mixing the recovered streams *(F₁)* and *(F₃)* with crude hydroquinone,
- concentrating the mixture obtained, resulting in the removal of a stream *(F₅)*,
- crystallizing the hydroquinone present in the concentrated stream,
- solid/liquid separation, making it possible to separate the crystalline hydroquinone from an aqueous phase composed of the crystallization aqueous mother liquors *(F₆)*,
- washing the separated hydroquinone, making it possible to collect aqueous wash liquors *(F₇)*,
- drying the washed hydroquinone with removal of water *(F₈),*
- mixing the recovered streams *(F₆)* and *(F₇)*,
- and recycling the mixture obtained to the stage of mixing the streams *(F₁)* and *(F₃)*.

6. Process according to Claim 5, **characterized in that** a bleeding is carried out at (P₃) on the aqueous phase *(F₆)* resulting from the separation of the hydroquinone recovered from the concentrated stream, before mixing with the stream *(F₇)* composed of the aqueous wash liquors.

7. Process according to one of Claims 1 to 6, **characterized in that** the dissolution is carried out by introduction of an amount of water such that the concentration of the hydroquinone in the water is between 20 and 50% by weight, preferably between 20 and 40% by weight.

8. Process according to Claim 7, **characterized in that** the dissolution operation is preferably carried out at a temperature ranging from 70°C to 100°C.

9. Process according to one of Claims 1 to 8, **characterized in that** the crystallization of the hydroquinone is carried out by cooling from the dissolution temperature down to a lower temperature of between 0°C and 40°C, preferably between 5°C and 25°C.

10. Process according to one of Claims 1 to 9, **characterized in that** the drying temperature is
advantageously chosen between 100°C and 140°C.

11. Process according to one of Claims 1 to 10, **characterized in that** the washing is carried out by employing an amount of water ranging from 20 to 60% of the weight of hydroquinone.

12. Process according to Claim 3, **characterized in that** the mixing of the streams *(F₁ₐ)* and *(F₃ₐ)* is carried out at a temperature advantageously chosen between 5°C and 70°C.

13. Process according to either of Claims 1 and 12, **characterized in that** the mixture obtained is concentrated so as to increase the concentration of hydroquinone in the medium from 5% by weight to 40% by weight, preferably from 7% to 35%.

14. Process according to Claim 13, **characterized in that** concentrating is carried out by reducing the pressure while remaining within the abovementioned temperature region; or by distilling at atmospheric pressure or at pressures lower than or greater than atmospheric pressure.

15. Process according to Claim 5, **characterized in that** the mixing of the streams *(F₆)* and *(F₇)* is carried out at a temperature advantageously chosen between 5°C and 70°C.

16. Process according to one of Claims 1 to 15, **characterized in that** the crude hydroquinone *HQ⁰* treated has contents of impurities of 0.5 to 4% by weight, preferably of 0.5 to 2% by weight and more preferably still of 1 to 2% by weight, with respect to the total weight of the crude hydroquinone.

17. Process according to one of Claims 1 to 16, **characterized in that** the crude hydroquinone *HQ⁰* treated comprises from 0.1 to 2% by weight, preferably from 0.2 to 1% by weight, of light impurities essentially composed of resorcinol and residual pyrocatechol.

18. Process according to one of Claims 1 to 17, **characterized in that** the crude hydroquinone *HQ⁰* treated comprises from 0.1 to 2% by weight, preferably from 0.2 to 1% by weight, of pyrogallol.

19. Process according to one of Claims 1 to 18, **characterized in that** the crude hydroquinone *HQ⁰* comprises, by weight with respect to the total amount of crude hydroquinone:
- from 96 to 99.5% of hydroquinone,
- from 0.1 to 2% of resorcinol,
- from 0.1 to 2% of pyrogallol,
- pyrocatechol in the form of traces at a content of less than 500 ppm (0.05%).

20. Process according to one of Claims 1 to 19, **characterized in that** the acid catalyst is chosen from:
- strong protic acids, preferably sulfuric acid, chlorosulfuric acid, perchloric acid or sulfonic acids, preferably methanesulfonic, trifluoromethanesulfonic, toluenesulfonic or phenolsulfonic acid,
- sulfonic resins, preferably a sulfonated styrene-divinylbenzene copolymer, a phenolformaldehyde copolymer which carries a methylenesulfonic group on the aromatic ring, or a perfluorinated resin carrying sulfonic groups,
- iron(II) and copper(II) complexes,
- acid zeolites, preferably zeolites of titanium silicalite (or titanosilicalite-1) type or iron silicalite type of TS-1 type, zeolites of MEL titanium silicalite type or titanozeosilites of MFI type, or MCM-22 zeolites.

21. Process according to Claim 1, **characterized in that** hydroquinone *HQ¹* is obtained, the characteristics of which are as follows:
- the hydroquinone content is greater than or equal to 98.5%,
- the resorcinol content is less than 4000 ppm,
- the pyrogallol content is less than 4000 ppm,
- the colouring is less than 500 Hazen.

22. Process according to Claim 2, **characterized in that** hydroquinone *HQ²* is obtained, the characteristics of which are as follows:
- the hydroquinone content is greater than or equal to 99.5%,
- the resorcinol content is less than 500 ppm,
- the pyrogallol content is less than 100 ppm,
- the colouring is less than 30 Hazen.

23. Process according to Claim 3, **characterized in that** hydroquinone *HQ³* is obtained, the characteristics of which are as follows:
- the hydroquinone content is greater than or equal to 99%,
- the resorcinol content is less than 5000 ppm,
- the pyrogallol content is less than 200 ppm,
- the colouring is less than 50 Hazen.

24. Process according to Claim 5, **characterized in that** hydroquinone *HQ⁴* is obtained, the characteristics of which are as follows:
- the hydroquinone content is greater than or equal to 99%,
- the resorcinol content is less than 5000 ppm,
- the pyrogallol content is less than 500 ppm,
- the colouring is less than 100 Hazen.

## Patentansprüche

1. Verfahren zur Herstellung von gereinigtem Hydrochinon ausgehend von einem Rohhydrochinon *HQ⁰,* das 96 bis 99,5 Gew.-% Hydrochinon und kleine Mengen von Verunreinigungen einschließlich 0,1 bis 2 Gew.-% Resorcin, 0,1 bis 2 Gew.-% Pyrogallol (wobei sich die Prozentangaben auf die Gesamtmengen von Rohhydrochinon beziehen) und Spuren von Brenzkatechin umfasst, **dadurch gekennzeichnet, dass** es mindestens die folgenden Schritte umfasst:
- Herstellen eines Gemischs, das 20 bis 80 Gew.-% Brenzkatechin, 80 bis 20 Gew.-% Hydrochinon, 0,1 bis 2 Gew.-% Resorcin, 0,1 bis 2 Gew.-% Pyrogallol umfasst, durch Hydroxylierung von Phenol mit Wasserstoffperoxid in Gegenwart eines sauren Katalysators,
- Destillieren des Gemischs zum Erhalt des Brenzkatechins am Kopf und des Rohhydrochinons am Sumpf,
- Lösen des Rohhydrochinons in Wasser,
- Kristallisieren des Hydrochinons,
- Abtrennen des gereinigten Hydrochinons
- und einen Schritt des Trocknens des gereinigten Hydrochinons.

2. Verfahren nach Anspruch 1, **dadurch gekennzeichnet, dass** es einen Waschschritt vor dem Trocknungsschritt umfasst.

3. Verfahren nach einem der Ansprüche 1 bis 2, **dadurch gekennzeichnet, dass** es die folgenden Schritte umfasst:
- Lösen des Rohhydrochinons in Wasser,
- Kristallisieren des Hydrochinons,
- Fest/Flüssig-Trennung, wodurch das kristallisierte Hydrochinon von einer wässrigen Phase, die aus den Kristallisationsmutterlaugen *(F₁ₐ)* besteht, abgetrennt werden kann,
- Waschen des abgetrennten Hydrochinons, wodurch Waschlaugen *(F₃ₐ)* gewonnen werden können,
- Trocknen des gewaschenen Hydrochinons unter Entfernung von Wasser *(F₄ₐ),*
- Mischen der gewonnenen Ströme *(F₁ₐ)* und *(F₃ₐ)*,
- Aufkonzentrieren des erhaltenen Gemischs
- und Zurückführen des Gemischs in den Auflösungs- oder Kristallisationsschritt.

4. Verfahren nach Anspruch 3, **dadurch gekennzeichnet, dass** man an (P₁), vorzugsweise am Ausgang der Aufkonzentrierungszone, oder an (P₂) an dem aus der wässrigen Phase *(F₁ₐ)* aus der Abtrennung des Hydrochinons bestehenden Strom nach dem Kristallisieren, aber vor dem Mischen mit dem aus den Waschlaugen bestehenden Strom *(F₃ₐ)* eine Ausschleusung durchführt.

5. Verfahren nach einem der Ansprüche 1 bis 2, **dadurch gekennzeichnet, dass** es die folgenden Schritte umfasst:
- Mischen der gewonnenen Ströme *(F₁ₐ)* und *(F₃ₐ)* mit Rohhydrochinon,
- Aufkonzentrieren des erhaltenen Gemischs, was zur Entfernung eines Stroms *(F₅)* führt,
- Kristallisieren des in dem aufkonzentierten Strom vorliegenden Hydrochinons,
- Fest/Flüssig-Trennung, wodurch das kristallisierte Hydrochinon von einer wässrigen Phase, die aus den Kristallisationsmutterlaugen *(F₆)* besteht, abgetrennt werden kann,
- Waschen des abgetrennten Hydrochinons, wodurch Waschlaugen *(F₇)* gewonnen werden können,
- Trocknen des gewaschenen Hydrochinons unter Entfernung von Wasser *(F₈),*
- Mischen der gewonnenen Ströme *(F₆)* und *(F₇)*,
- Aufkonzentrieren des erhaltenen Gemischs
- und Zurückführen des erhaltenen Gemischs in den Schritt des Mischens der Ströme *(F₁)* und *(F₃)*.

6. Verfahren nach Anspruch 5, **dadurch gekennzeichnet, dass** man an (P₃) an der wässrigen Phase *(F₆)* aus der Abtrennung des aus dem aufkonzentrierten Strom gewonnenen Hydrochinons vor dem Mischen mit dem aus den Waschlaugen bestehenden Strom *(F₇)* eine Ausschleusung durchführt.

7. Verfahren nach einem der Ansprüche 1 bis 6, **dadurch gekennzeichnet, dass** das Auflösen durch Eintragen einer solchen Wassermenge erfolgt, dass die Konzentration des Hydrochinons in dem Wasser zwischen 20 und 50 Gew.-% und vorzugsweise zwischen 20 und 40 Gew.-% liegt.

8. Verfahren nach Anspruch 7, **dadurch gekennzeichnet, dass** das Auflösen vorzugsweise bei einer Temperatur im Bereich von 70°C bis 100°C durchgeführt wird.

9. Verfahren nach einem der Ansprüche 1 bis 8, **dadurch gekennzeichnet, dass** man das Kristallisieren des Hydrochinons durch Abkühlen von der Auflösungstemperatur auf eine niedrigere Temperatur zwischen 0°C und 40°C und vorzugsweise zwischen 5°C und 25°C durchführt.

10. Verfahren nach einem der Ansprüche 1 bis 9, **dadurch gekennzeichnet, dass** die Trocknungstemperatur vorteilhafterweise zwischen 100°C und 140°C gewählt wird.

11. Verfahren nach einem der Ansprüche 1 bis 10, **dadurch gekennzeichnet, dass** man das Waschen unter Verwendung einer Wassermenge im Bereich von 20 bis 60% des Hydrochinongewichts durchführt.

12. Verfahren nach Anspruch 3, **dadurch gekennzeichnet, dass** man das Mischen der Ströme *(F₁ₐ)* und *(F₃ₐ)* bei einer vorteilhafterweise zwischen 5°C und 70°C gewählten Temperatur durchführt.

13. Verfahren nach einem der Ansprüche 1 bis 12, **dadurch gekennzeichnet, dass** man die erhaltene Mischung so aufkonzentriert, dass die Hydrochinonkonzentration in dem Medium von 5 Gew.-% auf 40 Gew.-% und vorzugsweise von 7% auf 35% erhöht wird.

14. Verfahren nach Anspruch 13, **dadurch gekennzeichnet, dass** man das Aufkonzentrieren durch Verringerung des Drucks unter Einhaltung des oben angegebenen Temperaturbereichs oder durch Destillation unter Normaldruck oder bei Drücken, die über oder unter Normaldruck liegen, durchführt.

15. Verfahren nach Anspruch 5, **dadurch gekennzeichnet, dass** man das Mischen der Ströme *(F₆)* und *(F₇)* bei einer vorteilhafterweise zwischen 5°C und 70°C gewählten Temperatur durchführt.

16. Verfahren nach einem der Ansprüche 1 bis 15, **dadurch gekennzeichnet, dass** das behandelte Rohhydrochinon *HQ⁰* Verunreinigungsgehalte von 0,5 bis 4 Gew.-%, vorzugsweise 0,5 bis 2 Gew.-% und noch weiter bevorzugt 1 bis 2 Gew.-%, bezogen auf das Gesamtgewicht des Rohhydrochinons, aufweist.

17. Verfahren nach einem der Ansprüche 1 bis 16, **dadurch gekennzeichnet, dass** das behandelte Rohhydrochinon *HQ⁰* 0,1 bis 2 Gew.-%, vorzugsweise 0,2 bis 1 Gew.-%, leichte Verunreinigungen, die im Wesentlichen aus Resorcin und restlichem Brenzkatechin bestehen, enthält.

18. Verfahren nach einem der Ansprüche 1 bis 17, **dadurch gekennzeichnet, dass** das behandelte Rohhydrochinon *HQ⁰* 0,1 bis 2 Gew.-%, vorzugsweise 0,2 bis 1 Gew.-%, Pyrogallol enthält.

19. Verfahren nach einem der Ansprüche 1 bis 18, **dadurch gekennzeichnet, dass** das Rohhydrochinon *HQ⁰,* bezogen auf das Gesamtgewicht des Rohhydrochinons,
- 96 bis 99,5% Hydrochinon,
- 0,1 bis 2% Resorcin,
- 0,1 bis 2% Pyrogallol,
- Brenzkatechin in Form von Spuren in einem Gehalt von weniger als 500 ppm (0,05%) enthält.

20. Verfahren nach einem der Ansprüche 1 bis 19, **dadurch gekennzeichnet, dass** der saure Katalysator ausgewählt wird aus:
- starken protischen Säuren, vorzugsweise Schwefelsäure, Chlorschwefelsäure, Perchlorsäure, Sulfonsäuren, vorzugsweise Methansulfonsäure, Trifluormethansulfonsäure, Toluolsulfonsäure, Phenolsulfonsäure,
- Sulfonsäureharzen, vorzugsweise einem sulfonierten Styrol-Divinylbenzol-Copolymer, einem Phenol-Formaldehyd-Copolymer mit einer Methylensulfongruppe am aromatischen Kern, einem perfluorierten Harz mit Sulfonsäuregruppen,
- Eisen-II- und Kupfer-II-Komplexen,
- sauren Zeolithen, vorzugsweise Zeolithen vom Titansilikalit-Typ (oder Titansilikalit-1-Typ) oder Eisensilikalit-Typ vom TS-1-Typ, Zeolithen vom MEL-Titansilikalit-Typ oder Titanzeosiliten vom MFI-Typ und MCM-22-Zeolithen.

21. Verfahren nach Anspruch 1, **dadurch gekennzeichnet, dass** man Hydrochinon *HQ¹* mit den folgenden Eigenschaften erhält:
- der Hydrochinongehalt ist größer gleich 98,5%,
- der Resorcingehalt ist kleiner als 4000 ppm,
- der Pyrogallolgehalt ist kleiner als 4000 ppm,
- die Färbung ist kleiner als 500 Hazen.

22. Verfahren nach Anspruch 2, **dadurch gekennzeichnet, dass** man Hydrochinon *HQ²* mit den folgenden Eigenschaften erhält:
- der Hydrochinongehalt ist größer gleich 99,5%,
- der Resorcingehalt ist kleiner als 500 ppm,
- der Pyrogallolgehalt ist kleiner als 100 ppm,
- die Färbung ist kleiner als 30 Hazen.

23. Verfahren nach Anspruch 3, **dadurch gekennzeichnet, dass** man Hydrochinon *HQ³* mit den folgenden Eigenschaften erhält:
- der Hydrochinongehalt ist größer gleich 99%,
- der Resorcingehalt ist kleiner als 5000 ppm,
- der Pyrogallolgehalt ist kleiner als 200 ppm,
- die Färbung ist kleiner als 50 Hazen.

24. Verfahren nach Anspruch 5, **dadurch gekennzeichnet, dass** man Hydrochinon *HQ⁴* mit den folgenden Eigenschaften erhält:
- der Hydrochinongehalt ist größer gleich 99%,
- der Resorcingehalt ist kleiner als 5000 ppm,
- der Pyrogallolgehalt ist kleiner als 500 ppm,
- die Färbung ist kleiner als 100 Hazen.
